(19) Europäisches Patentamt
European Patent Office
Office européen des brevets

(11) **EP 2 169 393 A1**

(12) **EUROPÄISCHE PATENTANMELDUNG**

(43) Veröffentlichungstag:
**31.03.2010 Patentblatt 2010/13**

(51) Int Cl.:
***G01N 27/08*** *(2006.01)*    ***G01N 33/18*** *(2006.01)*

(21) Anmeldenummer: **09405166.1**

(22) Anmeldetag: **24.09.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA RS**

(30) Priorität: **26.09.2008 EP 08405239**

(71) Anmelder: **R. Nussbaum AG**
**4601 Olten (CH)**

(72) Erfinder: **Gysin, Hans**
**6006 Luzern (CH)**

(74) Vertreter: **Rüfenacht, Philipp Michael et al**
**Keller & Partner**
**Patentanwälte AG**
**Schmiedenplatz 5**
**Postfach**
**3000 Bern 7 (CH)**

(54) **Verfahren und Vorrichtung zum Messen der Härte von Wasser**

(57)     Die Erfindung betrifft ein Verfahren zur Messung der Härte von Wasser sowie eine Vorrichtung zur Durchführung dieses Verfahrens. Die Bestimmung der Härte von Wasser erfolgt aus Messungen der elektrischen Leitfähigkeit des Wassers. Die Messungen der elektrischen Leitfähigkeit können durch das Anlegen eines Wechselstromes bei verschiedenen Frequenzen erfolgen. Des Weiteren kann die Wasserprobe in mindestens zwei Teilströme (2, 3, 4) aufgeteilt werden, wobei die einzelnen Teilströme (2, 3, 4) eine unterschiedliche Behandlung erfahren, die die elektrische Leitfähigkeit des jeweiligen Teilstroms ebenfalls beeinflusst. Auch wenn die Messung der elektrischen Leitfähigkeit alle im Wasser vorhandenen Ionen berücksichtigt, kann durch das erfindungsgemässe Verfahren die Konzentration derjenigen Ionen genauer gemessen werden, die vorwiegend zur Härte des Wassers beitragen. Dabei kann weitgehend auf Chemikalien verzichtet werden. Somit ist eine Vorrichtung zur Anwendung des erfindungsgemässen Verfahrens nahezu wartungsfrei.

# Fig. 1

EP 2 169 393 A1

**Beschreibung**

**Technisches Gebiet**

Die Erfindung betrifft ein Verfahren zur Messung der Härte von Wasser sowie eine Vorrichtung zur Durchführung dieses Verfahrens.

**Stand der Technik**

Es gibt viele Situationen, in denen die Kenntnis der Härte des Wassers hilfreich oder sogar notwendig ist. So zum Beispiel beim Einsatz von Reinigungsmitteln, deren benötigte Menge unter anderem auch von der Härte des Wassers abhängig ist. In diesem Zusammenhang werden seit einiger Zeit - besonders in Gebieten mit hartem Trinkwasser - vermehrt Entkalkungsanlagen eingesetzt.

Als Wasserhärte wird im Allgemeinen die Äquivalentkonzentration der im Wasser gelösten Ionen der Erdalkalimetalle, vor allem Calcium und Magnesium, bezeichnet. Die Bestimmung der Wasserhärte läuft somit auf eine Bestimmung der Äquivalentkonzentration dieser Ionen hinaus. Naturgemäss, wie in der Chemie üblich, erfolgt die Bestimmung im Labor am einfachsten und genausten mit einer Titrationsmethode. Tatsächlich schreiben die meisten offiziellen Methoden zur Bestimmung der Wasserhärte wie EPA 130.2 (US Environmental Protection Agency), ASTM D 511-93 (American Society for Testing and Materials) oder ISO 6059 eine Titrationsmethode vor. Meist wird als Titrant Ethylendiamintetraacetat (EDTA) verwendet.

Viele käufliche Wasserhärteanalysatoren bilden einen Laborprozess mit einem Gerät nach; es automatisiert den ganzen Ablauf - von der Probennahme bis zur Titration und fotometrischen Bestimmung des Farbumschlagpunktes. Ein Beispiel ist das Gerät "Stamolys CA 71 HA" der Firma Endress + Hauser AG, CH-4153 Reinach, oder das Gerät "Testomat 2000" der Firma CHRIST AQUA ecolife AG, CH-4147 Aesch. Diesen Geräten ist eine grosse Messgenauigkeit eigen, aber sie sind aufwendig und teuer. Überdies benötigen sie eine regelmässige Wartung - nicht zuletzt wegen der Verwendung eines Titranten.

Aus der Patentliteratur sind neben der Titrationsmethode viele andere Messverfahren für die Bestimmung der Wasserhärte bekannt. Grob lassen sie sich in die folgenden Kategorien einteilen:

- direkte oder indirekte Messung der Calcium - oder Magnesium-Ionenaktivität mittels ionenselektiven Elektroden (ISE), sowie

- Verfahren, die davon Gebrauch machen, dass Austauschharze entsprechend dem Fortschritt des Ionenaustauschs ihr Volumen, ihre Farbe oder ihre Dielektrizitätskonstante ändern.

- Messung der elektrischen Leitfähigkeit als Mass für die Wasserhärte.

Als Beispiel einer Messung der Härte von Wasser mittels ionenselektiver Sensorik sei die Druckschrift DE 198 54 651 (OFS Online Fluid Sensoric) erwähnt. Diese beschreibt ein Verfahren zum Betreiben einer Wasserenthärtungsanlage mit einer Regenerationseinheit durch eine in situ-Messung der Wasserresthärte mittels eines ionenselektiven Sensors. Einem Prozesswasser wird ein Teilstrom entnommen, der via eine Ventileinheit auf eine Sensoreinheit mit mindestens einem ionenselektiven Calcium -/Magnesium-Sensor gelenkt wird. Ionenselektive Sensoren müssen regelmässig kalibriert werden. Dazu kann über die Ventileinheit aus einem Kalibrierspeicher genau definierte Kalibrierflüssigkeit auf den Sensor gegeben werden. Des Weiteren umfasst die Sensoreinheit einen TemperaturSensor. Zusammen mit der Messung der Temperatur wird mit der Hilfe der ionenselektiven Sensoren die Calcium - sowie die Magnesium-Ionenkonzentration bestimmt. Bei zu hoher Konzentration wird ein Aktivierungssignal an die Regenerationseinheit der Wasserenthärtungsanlage gegeben. Ein Nachteil dieser Methode ist, dass die ionenselektiven Sensoren immer wieder mit einer Kalibrierflüssigkeit geeicht werden müssen. Dadurch erfordert eine Anlage, die nach einem solchen Verfahren funktioniert, einen technischen Mehraufwand bei der Konstruktion. Zudem sind solche Anlagen wartungsintensiv. Des Weiteren sind Methoden, die wie beschrieben ionenselektive Elektroden verwenden, wenig robust, weil es sich beim zentralen Prozess um einen Oberflächeneffekt handelt. Querempfindlichkeiten, Verschmutzung, Elektrodengifte etc. behindern den Einsatz dieser Verfahren in wartungsfreien Systemen.

Stellvertretend für Methoden, die Austauschharze verwenden, sei die Patentschrift DE 3406724 (Spiegl) erwähnt. Diese beschreibt einen Härtefühler mit einer regenerierbaren Füllung aus Ionen tauschendem Harz. Dabei wird ausgenutzt, dass die Füllung bei hartem Wasser zusammenschrumpft und sich beim Regenerieren wieder ausdehnt. Die Füllung befindet sich in einem zylindrischen Behälter, wobei genügend Raum für die Volumenänderung vorgesehen ist. Die Volumenänderung wird dann mit der Hilfe eines Kolbens, der durch eine Membrane getrennt auf der Füllung sitzt, gemessen. Dieser Kolben kann einen Schalter betätigen, der zum Beispiel die Regenerierungsphase des Ionentauschers auslöst. Methoden, die wie beschrieben Austauschharze verwenden, sind robuster, aber sie erlauben die Messung der absoluten Wasserhärte nicht. Sie können im Wesentlichen nur detektieren, ob ein vorgelagerter Ionentauscher erschöpft ist oder nicht. Austauschharze müssen wiederum regelmässig ausgewechselt bzw. regeneriert werden.

Damit verbleiben nur Verfahren, welche auf der Messung der elektrischen Leitfähigkeit des Wassers beruhen. Stellvertretend für diese Gattung sei das Dokument DE 195 37 059 A1 (Technische Universität Dresden) erwähnt. Darin wird eine Anordnung zum berührungslosen Messen der spezifischen Leitfähigkeit wässriger Lösungen offenbart. Die Anordnung besteht aus zwei Koppelelektroden, die kapazitiv einen Wechselstrom in eine Messzelle einkoppeln. Im elektrischen Ersatzschema der Anordnung wird die Messzelle durch eine parallel geschaltete Kapazität und einen Widerstand ersetzt. In Serie geschaltet befinden sich davor und danach die beiden Kapazitäten der Koppelektroden. Ein zur ganzen Anordnung in Serie geschalteter Referenzwiderstand dient zum Messen des Stromflusses durch die Messzelle. Dabei ist der Stromfluss proportional zur Konzentration der Härte bildenden Ionen in der wässrigen Lösung der Messzelle. Da die elektrische Leitfähigkeit stark von der Temperatur der wässrigen Lösung abhängt, wird der ermittelte Wert der elektrischen Leitfähig noch mit einer Temperaturfunktion korrigiert, damit auf die Härte der wässrigen Lösung geschlossen werden kann. Ein Nachteil dieser Methode ist, dass die Messung eine Summenmessung über alle in der wässrigen Lösung vorkommenden Ionenarten ist. Es gibt keine selektive Messung nur der zur Härte des Wassers beitragenden Calcium- und Magnesium-Ionen.

Vorrichtungen für die elektrischen Leitfähigkeitsmessungen können ziemlich robust ausgelegt werden, weil es sich beim Phänomen der elektrischen Leitfähigkeit um einen Volumeneffekt handelt. Allerdings sind diese Methoden in der Regel unspezifisch, weil zur elektrischen Leitfähigkeit unter anderem sowohl alle Kationen als auch alle Anionen in der Wasserprobe beitragen. Deshalb ist im Allgemeinen der Zusammenhang zwischen der elektrischen Leitfähigkeit und der Härte des Wassers schwach. Leitfähigkeitsmessungen, respektive Differenzmessungen der elektrischen Leitfähigkeit werden deshalb vornehmlich eingesetzt, um die Erschöpfung eines Ionentauschers zu detektieren: Wenn er erschöpft ist, dann werden die Calcium - und Magnesiumionen nicht mehr gegen Natriumionen ausgetauscht, was eine Änderung der Leitfähigkeit des Wassers nach sich zieht.

Die DE 41 14 380 A1 (Daimler-Benz) beschreibt ein Verfahren und eine Anordnung zur Bestimmung der Wasserhärte mittels Messung der Leitfähigkeit der Härte bildenden Ionen. Das Verfahren sieht vor, dass aus einem Zuflusskanal Wasser in einen Behälter mit einem Ionentauscher und danach in einen Behälter mit einem Anionentauscher geführt wird, bevor das Wasser weitergeleitet wird. Vor dem Kationentauscher, zwischen dem Ionentauscher und Anionentauscher und nach dem Anionentauscher sind jeweils zwei Messelektroden angeordnet, welche über einen Umschalter mit einem Messgerät verbunden sind, mit dem die elektrische Leitfähigkeit gemessen werden kann. Durch das Bestimmen der Differenz der Leitfähigkeit zwischen den Messelektroden wird die Härte des Wassers bestimmt.

Der Nachteil dieser Messanordnung ist, dass sie geeicht werden muss. Die Eichung wird vorgenommen, indem Wasserproben mit bekannter Härte eingelassen werden und die gemessenen Differenzen der elektrischen Leitfähigkeit den entsprechenden Härtewerten zugeordnet werden.

Eine Methode, die die Messung der elektrischen Leitfähigkeit im Hinblick auf die Bestimmung der Härte des Wassers spezifischer macht, wird in der Druckschrift DE 198 26 659 A1 (Wittmann) offenbart. Darin wird ein Prüfverfahren offenbart, mit dem man die Wirksamkeit von "physikalischen" Wasseraufbereitungsanlagen prüfen kann. Dabei wird von der Tatsache Gebrauch gemacht, dass sich der elektrische Leitwert des Wassers während des Erhitzens des Wassers ändert. Dieser Leitwert lässt sich als Funktion der Zeit darstellen. Wenn die Zeitfunktion von physikalisch aufbereitetem Wasser von derjenigen des unbehandelten Wassers abweicht, ist eine Beeinflussung des Wassers durch die physikalische Wasseraufbereitungsanlage nachgewiesen.

Das in DE 198 26 659 A1 (Wittmann) offenbarte Verfahren zeichnet sich im Wesentlichen durch drei Dinge aus:

i. Es wird nur eine behandelte mit einer unbehandelten Probe verglichen.

ii. Der Leitwert des Wassers muss während des Erhitzens dauernd gemessen werden, damit man den zeitlichen Verlauf des Leitwerts aufzeichnen und später mit dem anderen Zeitverlauf des Leitwerts vergleichen kann.

iii. Leitwertänderungen werden durch Erhitzen des Wassers verursacht.

Das gleichzeitige Behandeln der Wasserprobe, zum Beispiel durch Erhitzen, und das kontinuierliche Messen der elektrischen Leitfähigkeit respektive der Veränderung der elektrischen Leitfähigkeit auf Grund der Behandlung, ermöglicht nicht eine kontinuierliche Bestimmung der Härte des Wassers. Erst wenn die Messung der zeitlichen Änderung der elektrischen Leitfähigkeit als Folge des Erhitzens abgeschlossen ist, können die Daten verglichen und die Härte des Wassers bestimmt werden.

Aufgrund dessen lässt sich das beschriebene Verfahren auch nicht ohne Weiteres in Verschnittwasseranlagen einsetzen. Solche Anlagen umfassen im Wesentlichen eine Entkalkungsvorrichtung und ein Mischventil, wobei mit dem Mischventil entkalktes Wasser, welches zuvor mit der Entkalkungsvorrichtung entkalkt wurde, mit unbehandeltem Wasser verschnitten, wird um enthärtetes Wasser zu erhalten. Immer häufiger werden diese Verschnittwasseranlagen in Wasserleitungssystemen für den privaten Gebrauch aber auch in der Industrie eingesetzt. Sie mischen unbehandeltes Wasser derart mit enthärtetem Wasser, dass eine optimale Wasserhärte resultiert. Entsprechende Anlagen gewinnen immer mehr an Bedeutung, einerseits weil sich die Härte des unbehandelten Wassers infolge einer zunehmenden Anzahl von Wasserverbünden in kurzer Zeit in weiten Grenzen ändern kann, andererseits weil durch die Verschnittwasserregelung die Enthärtungsanlagen wirtschaftlicher betrieben werden können.

Weitere Verschnittwasseranlagen mit Vorrichtungen zur Messung der Härte von Wasser sehen vor, dass die Messvorrichtungen vorgängig mittels Wasser bekannter Härte kalibriert werden, damit aus der gemessenen elektrischen Leitfähigkeit die Härte von Wasser bestimmt werden kann. Das Kalibrieren von Messvorrichtung ist aber aufwändig und daher aus wirtschaftlicher Sicht teuer.

In der vorliegenden Druckschrift wird unter dem Begriff "unbehandeltes Wasser" Wasser verstanden, das noch keiner Behandlung unterzogen wurde, die die elektrische Leitfähigkeit des unbehandelten Wassers verändert. Mit "behandeltes Wasser" ist Wasser gemeint, das einer Behandlung unterzogen wurde, die die elektrische Leitfähigkeit des unbehandelten Wassers verändert hat. Unter "entkalktem Wasser" ist Wasser gemeint, welches einer Behandlung unterzogen wurde, welche dem Wasser die Härte bildenden Ionen vollständig bzw. nahezu vollständig entzieht. Mit "enthärtetes Wasser" ist Wasser gemeint, das durch Verschneiden von entkalktem Wasser und unbehandeltem Wasser entstanden ist.

## Darstellung der Erfindung

Aufgabe der Erfindung ist es, ein dem eingangs genannten technischen Gebiet zugehörendes Verfahren und eine Vorrichtung zur Messung der Härte von Wasser zu schaffen, wobei die Bestimmung der Härte des Wassers kontinuierlich und nahezu wartungsfrei erfolgen kann.

Die Lösung der Aufgabe ist durch die Merkmale des Anspruchs 1 definiert. Gemäss der Erfindung werden beim Verfahren zur Bestimmung der Härte von Wasser folgende Schritte ausgeführt:

a) Bestimmen mindestens zweier Werte der elektrischen Leitfähigkeit der Wasserprobe unter verschiedenen Bedingungen und

b) Bestimmen der Härte des Wassers der Wasserprobe aus den mindestens zwei Werten der elektrischen Leitfähigkeit.

Zur elektrischen Leitfähigkeit tragen alle im Wasser gelösten Anteile bei, das heisst sowohl Anionen als auch Kationen. Indem zwei Werte der elektrischen Leitfähigkeit unter verschiedenen Bedingungen gemessen werden, ist es möglich, jene Anteile des Wassers, das heisst insbesondere die Konzentrationen der Magnesium- und der Calcium-Ionen, genauer zu bestimmen, welche vorwiegend für die Härte des Wassers bestimmend sind. Verschiedene Bedingungen zur Messung der elektrischen Leitfähigkeit können zum Beispiel dadurch gegeben sein, dass die Wasserprobe unterschiedlich behandelt, zum Beispiel erhitzt wird, wodurch sich die elektrische Leitfähigkeit des Wassers ändert. Es ist auch möglich, dass die unterschiedlichen Bedingungen dadurch gegeben sind, dass die elektrische Leitfähigkeit unterschiedlich gemessen wird, zum Beispiel indem die (komplexe) Impedanz des Wassers bei unterschiedlichen Frequenzen bestimmt wird. Ein weiterer Vorteil ist, dass eine kontinuierliche Messung der elektrischen Leitfähigkeit und daher eine kontinuierliche Bestimmung der Härte des Wassers möglich ist. Dadurch erhält man den Vorteil, dass bei einem Verschneiden von entkalktem Wasser und unbehandeltem, kalkhaltigem Wasser Schwankungen im Härtegrad des Wassers in situ gemessen werden können und das Verschneiden dementsprechend angepasst werden kann. Das erfindungsgemässe Verfahren ist somit insbesondere für die Verwendung mit Verschnittwasseranlagen geeignet. Indem die elektrische Leitfähigkeit gemessen wird, kann weitestgehend auf Chemikalien verzichtet werden. Daher erübrigt sich auch das periodische Nachfüllen von Chemikalien in Anlagen, die nach dem erfindungsgemässen Verfahren arbeiten. Dies macht eine Anlage nahezu wartungsfrei.

Eine Vorrichtung zur Bestimmung der Härte eines Wasserstroms nach dem erfindungsgemässen Verfahren besteht aus folgenden Teilen:

a) Mindestens einer Messvorrichtung zum Messen der elektrischen Leitfähigkeit des Wasserstroms,

b) einer Auswertungseinheit zum Auswerten von mindestens zwei, unter verschiedenen Bedingungen vorgenommenen Messungen der elektrischen Leitfähigkeit zum Bestimmen der Härte des Wassers.

Dabei ist es möglich, dass mit derselben Messvorrichtung zwei verschiedene Werte der elektrischen Leitfähigkeit gemessen werden, indem die Messvorrichtung die einzelnen Ionen-Anteile im Wasser, die zur elektrischen Leitfähigkeit beitragen (z. B. beteiligte Ionensorten), unterschiedlich berücksichtigt. Es ist zum Beispiel möglich, dass die Messvorrichtung die (komplexe) Impedanz des Wassers für unterschiedliche Frequenzen misst. Alternativ können mehrere Messvorrichtungen vorgesehen sein, die jeweils einen der Werte liefern.

Mit Vorteil gehen den oben beschriebenen Verfahrensschritten folgende Schritte voraus:

a) Aufteilen der Wasserprobe in mindestens zwei Fraktionen,

b) Unterziehen mindestens einer der Fraktionen einer Behandlung, welche die elektrische Leitfähigkeit des Wassers beeinflussen kann.

Die Bestimmung der Werte der elektrischen Leitfähigkeit des Wassers wird dann an mindestens zwei der Fraktionen vorgenommen.

Die Behandlung ist so gewählt, dass sie die einzelnen Anteile des Wassers, die zur elektrischen Leitfähigkeit beitragen (z. B. beteiligte Ionensorten), unterschiedlich beeinflusst. Somit ergeben sich durch die Behandlung unterschiedliche Werte für die elektrische Leitfähigkeit der einzelnen Fraktionen, die Aufschluss über die verschiedenen härterelevanten Anteile des Wassers geben. Dadurch sind bessere Rückschlüsse möglich, wie sich das Wasser zusammensetzt und somit kann eine genauere Angabe zur Härte des Wassers gemacht werden. In je mehr Fraktionen eine Wasserprobe aufgeteilt und unterschiedlichen Behandlungen unterworfen wird, desto genauer lässt sich im Prinzip die Härte des Wassers bestimmen.

Mit Vorteil wird dazu die Vorrichtung zur Bestimmung der Härte des Wassers durch folgende Einrichtungen ergänzt:

a) Eine Verteilstation zum Trennen des Wasserstroms in mindestens zwei Teilströme,

b) mindestens eine Behandlungsvorrichtung zur Beeinflussung der elektrischen Leitfähigkeit des Wassers eines Teilstroms, insbesondere ein kontinuierlicher Reaktor (Konti-Reaktor) und

c) mehrere Messvorrichtungen zum Messen der Leitfähigkeit der Teilströme.

Die Verteilstation ermöglicht das Fraktionieren der Wasserprobe. Die einzelnen Teilströme werden dann in den jeweiligen Behandlungsvorrichtungen behandelt, wodurch die elektrische Leitfähigkeit der Teilströme unterschiedlich verändert wird. Die Behandlungsvorrichtungen können aus kontinuierlichen Reaktoren (Konti-Reaktoren) bestehen, in denen die Teilströme der jeweils entsprechenden Behandlung unterworfen werden. Daraus ergibt sich der Vorteil, dass durch die verschiedenen Messresultate der elektrischen Leitfähigkeit ein genauerer Rückschluss auf die Zusammensetzung der einzelnen im Wasser gelösten Ionen-Anteile möglich ist. Dies wiederum ermöglicht eine präzisere Bestimmung der Härte des Wassers. Zudem kann die Behandlungsvorrichtung zeitlich aber auch örtlich von der Messvorrichtung getrennt sein. Dies hat wiederum den Vorteil, dass in situ gemessen werden kann. Das heisst, die einzelnen Teilströme fliessen je durch eine Behandlungsvorrichtung und danach durch mindestens eine Messvorrichtung. Dabei wird die elektrische Leitfähigkeit der einzelnen Teilströme gleichzeitig gemessen. Zur gleichen Zeit fliesst weiteres Wasser der Teilströme durch die Behandlungsvorrichtungen.

Alternativ ist es auch möglich, unterschiedliche Methoden zum Messen der elektrischen Leitfähigkeit an einer Wasserprobe vorzunehmen, ohne diese aufzuspalten. Es ist ebenfalls möglich, nicht alle Teilströme durch eine Behandlungsvorrichtung hindurchzuführen. Zudem ist auch vorstellbar, dass die Messung der elektrischen Leitfähigkeit im Rahmen des erfindungsgemässen Verfahrens zeitgleich während der Behandlung in der Behandlungsvorrichtung durchgeführt wird.

Alternativ ist es möglich, an dem nicht fraktionierten Wasser die elektrische Leitfähigkeit mittels zweier (oder mehrerer) Verfahren zu messen, wobei die Verfahren die verschiedenen Anteile im Wasser unterschiedlich berücksichtigen. Dadurch erhält man ebenfalls mindestens zwei Werte für die elektrische Leitfähigkeit, woraus sich wiederum die Härte des Wassers zuverlässig bestimmen lässt.

Mit Vorteil bleibt eine der mindestens zwei Fraktionen der Wasserprobe unbehandelt. Dadurch wird eine geringere Anzahl Behandlungen notwendig, was zu einer Vereinfachung des Verfahrens führt.

Alternativ können alle Fraktionen der Wasserprobe einer Behandlung unterzogen werden. Zudem ist vorstellbar, dass die Wasserproben zwar in einzelne Fraktionen aufgeteilt werden, aber keiner Behandlung unterzogen werden. Mehrere aussagekräftige Werte der elektrischen Leitfähigkeit können dann dadurch gewonnen werden, dass die elektrische Leitfähigkeit der einzelnen Fraktionen mit unterschiedlichen Verfahren gemessen wird.

Mit Vorteil erfolgt die Behandlung mindestens einer Fraktion physikalisch, insbesondere durch Erhitzen, oder chemisch. Insbesondere ist auch eine elektro-chemische oder katalytische Behandlung möglich, wie sie in DE 10 2007 002 016 A1 (Sasserath) respektive EP 0 957 066 A1 (Maitron) beschrieben werden. Unterschiedliche Verfahren verändern dabei die verschiedenen Anteile des Wassers, die zur elektrischen Leitfähigkeit des Wassers beitragen. Dies wiederum lässt Rückschlüsse auf die Härte des Wassers zu. Dabei ist es möglich, dass einzelne Fraktionen auf unterschiedliche Temperaturen erhitzt werden oder dass physikalische sowie chemische, insbesondere elektro-chemische Behandlungen miteinander kombiniert werden. So kann zum Beispiel ein Teil der Fraktionen auf unterschiedliche Temperaturen erhitzt werden und der andere Teil kann einer elektro-chemischen Behandlung unterworfen werden. Auch können nur physikalische oder nur chemische Behandlungen angewendet werden.

Mit Vorteil werden mittels einer ersten Messung an unbehandeltem Wasser ein erster elektrischen Leitfähigkeitswert $\sigma_{CaH2O}$ und mittels einer zweiten Messung an behandeltem Wasser ein zweiter elektrischen Leitfähigkeitswert $\sigma_{NaH2O}$ bestimmt. Dabei wird das unbehandelte Wasser durch einen Ionentauscher behandelt, um entkalktes, behandeltes Wasser zu erhalten. Die zweite Messung erfolgt dann an dem entkalkten, behandelten Wasser. Der Ionentauscher tauscht Calcium-Ionen durch Natrium-Ionen aus, womit die elektrische Leitfähigkeit des Wassers verändert wird. Diese Änderung ist dann mit der zweiten Messung feststellbar. Das beschriebene Verfahren hat den Vorteil, dass nur genau zwei Messungen der elektrischen Leitfähigkeit und eine Behandlung des Wassers notwendig sind. Zudem hat der Ionentauscher zum Behandeln des Wassers den Vorteil, dass danach entkalktes Wasser vorhanden ist, das im späteren Gebrauch wieder mit unbehandeltem Wasser verschnitten werden kann, um enthärtetes Wasser einer gewünschten Härte zu erhalten.

Alternativ sind andere Behandlungsmethoden, wie zum Beispiel Erhitzen des Wassers, anwendbar, die die elektrische Leitfähigkeit des Wassers ebenfalls beeinflussen.

Mit Vorteil wird aus den beiden ermittelten elektrischen Leitfähigkeitswerten $\sigma_{CaH2O}$ und $\sigma_{NaH2O}$ mittels der Beziehung

$$c_{Ca} = (\sigma_{NaH2O} - \sigma_{CaH2O})/(2 * \Lambda o_{Na} - \Lambda o_{Ca}) \qquad (1)$$

eine Konzentration $c_{Ca}$ von Calcium-Ionen des unbehandelten Wassers ermittelt, wobei $\Lambda o_{Ca}$ die Grenzleitfähigkeit von Calcium bei 25°C ist und $\Lambda o_{Na}$ die Grenzleitfähigkeit von Natrium bei 25°C ist. Ist der Beitrag der Magnesium-Ionen an die Härte des zu bestimmenden Wassers vernachlässigbar, so kann aus der Konzentration $c_{Ca}$ von Calcium-Ionen ohne weiteres die Härte von Wasser abgeleitet werden. Zum Beispiel kann mittels der bekannten Beziehung $1°fH = 0.2 * c_{CA}$ die Härte von Wasser in Einheiten der französischen Härtegrade angegeben werden, wobei die Einheit von $c_{CA}$ $10^{-3}$ Mol pro Liter [mmol/l] bzw. Mol pro Kubik-Meter [mol/m$^3$] ist. Der Beitrag der Magnesium-Ionen an die Härte des Wassers ist insbesondere für natürlich vorkommendes Wasser, insbesondere Trinkwasser, vernachlässigbar. Der Vorteil der angegebenen Beziehung besteht darin, dass eine einfache Beziehung zwischen den bestimmten elektrischen Leitfähigkeitswerten $\sigma_{CaH2O}$ und $\sigma_{NaH2O}$ und der Konzentration $c_{Ca}$ von Calcium-Ionen angegeben werden kann. Insbesondere ist durch Anwendung der Beziehung kein vorgängiges Kalibrieren mehr notwendig, was eine erhebliche Vereinfachung des Verfahrens bedeutet.

Alternativ könnten die bestimmten elektrischen Leitfähigkeitswerte $\sigma_{CaH2O}$ und $\sigma_{NaH2O}$ mit vorher kalibrierten Werten verglichen werden und daraus die Konzentration $c_{Ca}$ von Calcium-Ionen bestimmt werden.

Mit Vorteil wird eine Wassertemperaturdifferenz zwischen der ersten Messung und der zweiten Messung berücksichtigt, indem der gemessene elektrische Leitfähigkeitswert $\sigma_{CaH2O}$ von unbehandeltem Wasser und/oder der gemessene elektrische Leitfähigkeitswert $\sigma_{NaH2O}$ von entkalktem Wasser nach dem Ionentauscher entsprechend korrigiert wird. Da der Durchlauf durch den Ionentauscher die Temperatur des Wassers beeinflussen, insbesondere erhöhen, kann, wird durch die Berücksichtigung der Wassertemperaturdifferenz die Auswirkung der Temperaturänderung auf das Ergebnis der Härtebestimmung korrigiert. In der DIN-Norm DIN EN 27888: 1993 ist ausführlich beschrieben, wie die Messung der elektrischen Leitfähigkeit von der Temperatur abhängt. Zudem wird in der erwähnten DIN-Norm beschrieben, wie ein gemessener elektrischer Leitfähigkeitswert von Wasser, welcher bei einer bestimmten Temperatur gemessen wurde, auf eine Normtemperatur von 25,0°C korrigiert werden kann, damit dieser normierte elektrische Leitfähigkeitswert mit anderen normierten elektrischen Leitfähigkeitswerten verglichen werden kann.

Es gibt verschiedene Möglichkeiten die Wassertemperaturdifferenz zu berücksichtigen. Zum Beispiel ist es möglich, dass jeweils für die erste und die zweite Messung des elektrischen Leitfähigkeitswerts zugleich auch eine erste und eine zweite Wassertemperatur bestimmt werden und dann die gemessenen elektrischen Leitfähigkeitswerte gemäss der DIN-Norm DIN EN 27888 : 1993 auf die Normtemperatur von 25,0°C korrigiert werden.

Bei einer alternativen Ausführungsform wird mittels einer Temperaturänderungsvorrichtung eine Temperatur des entkalkten Wassers derart verändert, dass die Temperatur des entkalkten Wassers während der zweiten Messung des elektrischen Leitfähigkeitswerts einer Temperatur des unbehandelten Wassers entspricht. Aufgrund der Tatsache, dass die lineare Abhängigkeit der elektrischen Leitfähigkeit von der Temperatur für unterschiedliche Ionen-Konzentrationen im Wasser annähernd gleich ist, ist es nicht notwendig, dass für die erste und die zweite Messung das Wasser auf die Normtemperatur von 25,0°C gemäss der DIN-Norm DIN EN 27888 gebracht wird. Für die Gültigkeit der Beziehung (1) ist es ausreichend, dass die erste Messung und die zweite Messung der elektrischen Leitfähigkeit bei gleicher Wassertemperatur stattfinden. Dies hat den Vorteil, dass keine Korrektur der gemessenen elektrischen Leitfähigkeitswerte notwendig ist. Zudem hat das Angleichen der Temperatur des entkalkten Wassers an die Temperatur des unbehandelten Wassers den Vorteil, dass bei einem allfälligen nachfolgenden Verschneiden von unbehandeltem und entkalktem Wasser trotz unterschiedlichen Verschnittverhältnissen keine Temperaturschwankungen auftreten.

Die rechnerische Korrektur der elektrischen Leitfähigkeit auf Grund der Temperaturabhängigkeit und die Anpassung der Temperatur des entkalkten Wassers für die zweite Messung sind zwei Möglichkeiten, die Temperaturabhängigkeit der elektrischen Leitfähigkeit von Wasser zu berücksichtigen.

Ablagerungen, die zum Beispiel durch Verunreinigungen im Wasser oder auf Grund der Behandlung der einzelnen Fraktionen entstehen, können die Messung der elektrischen Leitfähigkeit beeinträchtigen. Mit Vorteil werden deshalb Ablagerungen, insbesondere solche, die auf Grund der Behandlungen der einzelnen Fraktionen entstehen, mittels entsprechender und an sich bekannter Methoden, z. B. mittels Ultraschall, periodisch und automatisch entfernt. Dadurch wird die Genauigkeit der Messungen der elektrischen Leitfähigkeit bewahrt.

Ebenfalls von Vorteil ist es, wenn die Entfernung der Ablagerungen permanent erfolgt, z. B. durch die permanente Erzeugung von Ultraschallschwingungen. Inwieweit Ablagerungen periodisch oder permanent entfernt werden, wird in Abhängigkeit davon bestimmt, mit welchen Ablagerungen zu rechnen ist und welche Genauigkeit für die Messung der elektrischen Leitfähigkeit gefordert ist.

Mit Vorteil sind somit an den Teilströmen Reinigungseinheiten angebracht, mit deren Hilfe Ablagerungen entfernt werden können. Insbesondere können solche Reinigungseinheiten direkt an den Behandlungsvorrichtungen angebracht sein.

Mit Vorteil handelt es sich bei diesen Reinigungseinheiten um Ultraschallgeräte, mit deren Hilfe Ablagerungen periodisch und/oder permanent entfernt werden können. Ein Ultraschallgerät ist ein effektives und einfaches Gerät zum Entfernen von Ablagerungen, das die nachfolgende Messvorrichtung nicht beeinträchtigt.

Alternativ sind auch andere Reinigungseinheiten denkbar, die Ablagerungen zum Beispiel mechanisch, elektrochemisch oder durch die Einwirkung elektro-magnetischer Strahlung entfernen.

Alternativ, kann auch auf eine Entfernung der Ablagerungen und auf entsprechende Reinigungseinheiten verzichtet werden. Zudem können zum Beispiel Filter oder vorgeordnete Reinigungseinheiten vorgängig Verunreinigungen in der Wasserprobe entfernen, so dass keine Ablagerungen entstehen.

Mit Vorteil wird das zu messende, unbehandelte Wasser einer Vorbehandlung unterzogen, so dass eine Ablagerung an einer Messsonde zur Messung der elektrischen Leitfähigkeit minimiert wird, ohne dass die Härte des unbehandelten Wassers verändert wird. Die Minimierung der Ablagerung an der Messsonde hat den Vorteil, dass die Genauigkeit der Messung der elektrischen Leitfähigkeit erhöht wird. Eine mögliche Vorbehandlung kann die Bildung von Kalk-Clustern durch den Einsatz elektrischer Felder sein. Dabei vereinen sich die einzelnen Calcium-Ionen zu Clustern, die zwar nach wie vor im Wasser gelöst sind, sich aber aufgrund der Clusterbildung kaum an der Messsonde ablagern.

Alternativ können Messverfahren zur Messung der elektrischen Leitfähigkeit verwendet werden, die durch Kalkablagerungen an Messsonden nicht beeinträchtigt werden.

Mit Vorteil umfasst die Vorrichtung zur Bestimmung der Härte eines Wasserstroms nach dem erfindungsgemässen Verfahren eine erste Messvorrichtung zur Messung eines elektrischen Leitfähigkeitswerts von unbehandeltem Wasser, einen Ionentauscher zur Behandlung von Wasser und eine zweite Messvorrichtung zur Messung eines elektrischen Leitfähigkeitswerts von behandeltem, entkalktem Wasser nach dem Ionentauscher. Dies ermöglicht einen einfachen und kostengünstigen Aufbau zur Messung der Härte von Wasser, ohne dass vorgängig eine aufwändige Kalibrierung der Vorrichtung notwendig ist. Von Vorteil ist auch, dass die Vorrichtung ein kontinuierliches Messen der Härte von Wasser erlaubt.

Alternative Anordnungen ergeben sich zum Beispiel daraus, dass nur eine Messvorrichtung vorhanden ist. Damit zwei unterschiedliche elektrische Leitfähigkeitswerte bestimmt werden können, wird der Wasserstrom aufgeteilt, wobei nur einen Teil den Ionentauscher durchfliesst. Der Messvorrichtung wird dann mittels Ventilen abwechslungsweise unbehandeltes Wasser und behandeltes, entkalktes Wasser zugeführt.

Die Auswertungseinheit bestimmt dann aus den bestimmten elektrischen Leitfähigkeitswerten $\sigma_{CaH2O}$ und $\sigma_{NaH2O}$ mit Hilfe der Beziehung (1) die Härte des unbehandelten Wassers.

Bei bevorzugten Ausführungsformen der Erfindung erfolgt die Messung der elektrischen Leitfähigkeit mittels Anlegen eines Wechselsignals, insbesondere eines Wechselstroms, bei verschiedenen Frequenzen. Da die einzelnen im Wasser gelösten Ionen-Anteile bei verschiedenen Frequenzen unterschiedlich zur elektrischen Leitfähigkeit beitragen, ist es möglich, genauer auf die einzelnen Ionenkonzentrationen des Wassers zu schliessen und somit die Härte des Wassers zu bestimmen. Die Verwendung unterschiedlicher Frequenzen ist somit eine einfache und schnelle Methode, die elektrische Leitfähigkeit zu messen. Des Weiteren bietet das Anlegen eines Wechselsignals den Vorteil, dass durch das Messen der elektrischen Leitfähigkeit bei verschiedenen Frequenzen verschiedene aussagekräftige Werte gewonnen werden können.

Zur Durchführung dieses Verfahrens besteht die Messvorrichtung aus einer Einheit, die ein Wechselsignal, insbesondere einen Wechselstrom, bei verschiedenen Frequenzen erzeugt, wodurch sich die elektrische Leitfähigkeit ermitteln lässt. Es ist möglich, die Leitfähigkeit eines Teilstromes für verschiedene Frequenzen mit der gleichen Messvorrichtung zu messen.

Alternativ sind andere Messungen der elektrischen Leitfähigkeit, zum Beispiel das Anlegen einer Gleichspannung zwischen Elektroden, vorstellbar, wonach der Stromfluss durch einen Teilstrom gemessen und so auf die elektrische Leitfähigkeit geschlossen wird.

Wie erwähnt ist das erfindungsgemässe Verfahren besonders zum Einsatz mit Verschnittwasseranlagen geeignet. Das heisst, vorgängig wird ein Verschneiden von entkalktem Wasser mit unbehandeltem, kalkhaltigem Wasser durchgeführt. Mit Hilfe des Verfahrens kann die Härte des Wassers nach dem Verschneiden bestimmt werden. Allfällige Schwankungen der Härte des Wassers werden dadurch sofort erkannt und das Mischverhältnis von entkalktem und kalkhaltigem Wasser kann sofort korrigiert werden. Dies ist von besonderem Vorteil, wenn die Härte des unbehandelten Wassers massiv schwankt, z. B. weil die Trinkwasserversorgung Wasser unterschiedlichen Ursprungs (z. B. Quellwasser, Seewasser, Grundwasser) fördert. Indem die Härte des Wassers nach dem Verschneiden bestimmt wird, kann ebenfalls erkannt werden, wenn eine vorgeordnete Entkalkungsanlage, die das Wasser zum Verschneiden entkalkt, erschöpft ist oder nicht mehr ordnungsgemäss arbeitet.

Alternativ kann zuerst die Härte des Wassers bestimmt werden und danach erst das Verschneiden des Wassers erfolgen. Das Mischverhältnis wird auf Grund der Härte des kalkhaltigen Wassers gewählt. Des Weiteren kann das Verfahren zum Bestimmen der Härte des Wassers auch bei anderen Anwendungen zum Einsatz kommen. So zum Beispiel bei Waschmaschinen, die auf Grund der bestimmten Wasserhärte die Menge eines Reinigungsmittels festlegen.

Mit Vorteil wird Wasser enthärtet, indem unbehandeltes Wasser zuerst entkalkt wird und danach mit unbehandeltem Wasser zu enthärtetem Wasser verschnitten wird. Dabei wird in zeitlichen Abständen eine Härte des unbehandelten Wassers nach einem der vorher beschriebenen Verfahren bestimmt. Mit der Hilfe der bestimmten Härte des unbehandelten Wassers wird ein Mischverhältnis von unbehandeltem Wasser und entkalktem Wasser derart eingestellt, dass eine bestimmte Härte des enthärteten Wassers erhalten wird. Die zeitlichen Abstände zwischen der Bestimmung der Härte von Wasser können je nach den zu erwartenden Schwankungen der Härte des Wassers angepasst werden. So kann der zeitliche Abstand zum Beispiel gleich Null sein, das heisst es wird kontinuierlich gemessen. Der zeitliche Abstand kann aber auch Minuten, Stunden oder sogar einen Tag oder mehrere Tage betragen.

Mit Vorteil umfasst eine Anordnung zum Enthärten von Wasser eine Entkalkungsvorrichtung zum Entkalken von Wasser, eine Verschnittvorrichtung zum Verschneiden von unbehandeltem Wasser mit entkalktem Wasser zu enthärtetem Wasser und eine vorher beschriebene, erfindungsgemässe Vorrichtung zur Bestimmung der Härte von unbehandeltem Wasser. Dabei wird die Verschnittvorrichtung mittels der Vorrichtung zur Bestimmung der Härte des unbehandelten Wassers derart eingestellt, dass eine bestimmte Härte des enthärteten Wassers erhalten wird.

Aus der nachfolgenden Detailbeschreibung und der Gesamtheit der Patentansprüche ergeben sich weitere vorteilhafte Ausführungsformen und Merkmalskombinationen der Erfindung.

## Kurze Beschreibung der Zeichnungen

Die zur Erläuterung des Ausführungsbeispiels verwendeten Zeichnungen zeigen:

Fig. 1    eine schematische Darstellung der Teilschritte eines ersten erfindungsgemässen Verfahrens zur Bestimmung der Härte von Wasser;

Fig. 2    eine schematische Darstellung der Teilschritte eines zweiten erfindungsgemässen Verfahrens zur Bestimmung der Härte von Wasser;

Fig. 3    eine schematische Darstellung der Teilschritte eines dritten erfindungsgemässen Verfahrens zur Bestimmung der Härte von Wasser;

Fig. 4    eine schematische Darstellung der Teilschritte eines vierten erfindungsgemässen Verfahrens zur Bestimmung der Härte von Wasser; und

Fig. 5    eine schematische Darstellung der Teilschritte eines fünften erfindungsgemässen Verfahrens zur Bestimmung der Härte von Wasser;

Fig. 6    eine schematische Darstellung des Einsatzes einer erfindungsgemässen Vorrichtung in einer ersten geregelten Verschnittwasseranlage;

Fig. 7    eine schematische Darstellung des Einsatzes einer erfindungsgemässen Vorrichtung in einer zweiten geregelten Verschnittwasseranlage; und

Fig. 8    eine schematische Darstellung des Einsatzes einer erfindungsgemässen Vorrichtung in einer dritten geregelten Verschnittwasseranlage.

Grundsätzlich sind in den Figuren gleiche Teile mit gleichen Bezugzeichen versehen.

**Wege zur Ausführung der Erfindung**

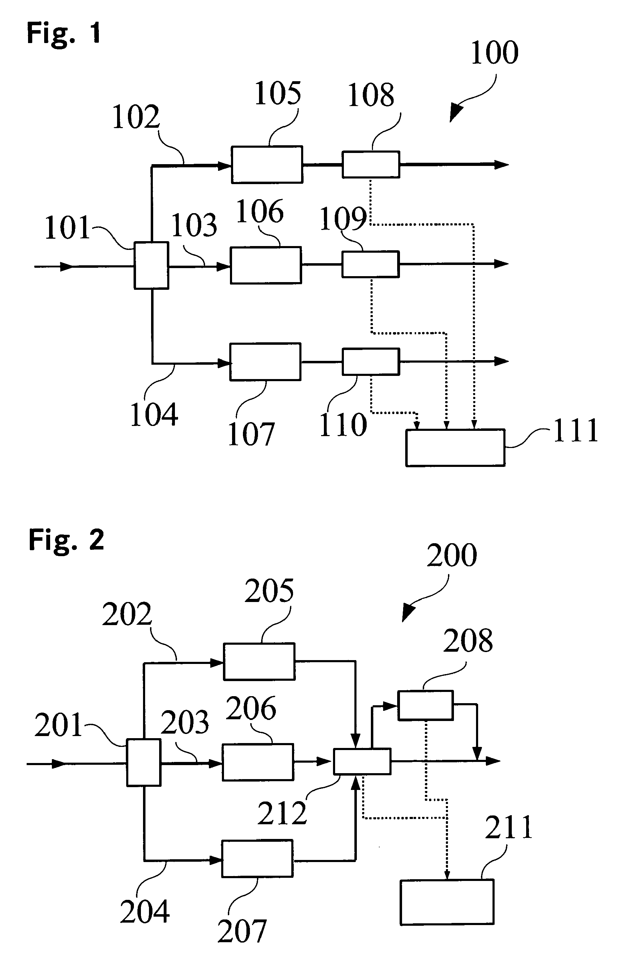

Die Figur 1 zeigt die verschiedenen Schritte einer ersten erfindungsgemässen Verfahrensvariante 100. Zuerst fliesst Wasser, dessen Härte bestimmt werden soll, in eine Verteilstation 1. Diese teilt das Wasser in drei Teilströme (Fraktionen) 102, 103 und 104 auf. Jeder dieser Teilströme wird dann einer Behandlung unterzogen. Der Teilstrom 102 durchläuft einen Kühler-Erhitzer 105, der die Temperatur des Wassers auf einen Standardwert, zum Beispiel 20°C, einstellt. Der Teilstrom 103 durchläuft einen Erhitzer 106; er erwärmt das Wasser auf eine Temperatur von 70°C, bei der ein Teil des Calciums in Form von Kalk ausgeschieden wird. Der Teilstrom 104 durchläuft einen zweiten Erhitzer 107, welcher das Wasser auf eine Temperatur von 100 °C erwärmt, so dass ein grosser Teil des Calciums in Form von Kalk ausgeschieden wird.
Die nachfolgenden Leitfähigkeitsmesseinrichtungen 108, 109 und 110 messen folglich unterschiedliche Werte für die elektrische Leitfähigkeit. Die Auswertung der drei Werte in einem Auswertegerät 111 lässt deshalb einen Rückschluss auf den ursprünglichen Gehalt der Calcium-Ionen im unbehandelten Wasser zu und somit kann die Härte des Wassers bestimmt werden.
Allfällige Kalkablagerungen in den Erhitzern 105, 106 und 107 lassen sich rein physikalisch entfernen, zum Beispiel durch starke akustische Einwirkung in Form von Ultraschall. Allenfalls kann die akustische Einwirkung dauerhaft und so stark gewählt werden, dass sich der Kalk gar nicht an den Wänden der Erhitzer ablagern kann, sondern im Wasser in Form von Mikropartikeln ausfällt. Diese werden vom Wasserstrom mitgetragen, tragen aber in dieser Form praktisch nichts mehr zur Leitfähigkeit bei.
Die Figur 2 zeigt eine mögliche Abfolge der verschiedenen Schritte einer zweiten erfindungsgemässen Verfahrensvariante 200. Analog wie bei der ersten Verfahrensvariante 100 wird das zu messende Wasser in einer Verteilstation 202 auf verschiedene Teilströme 202, 203 und 204 aufgeteilt und auf die verschiedenen Behandlungseinrichtungen 205, 206 und 207 gelenkt. Bei dieser Verfahrensvariante 200 wird im Unterschied zur ersten Verfahrensvariante 100 aber nur eine Leitfähigkeitsmesseinrichtung 208 eingesetzt. Nach der Behandlung der Teilströme 205, 206 und 207 werden diese nämlich mittels Ventile einer zweiten Verteilstation 212 abwechslungsweise auf die Leitfähigkeitsmesseinrichtung 208 gelenkt, wo der entsprechende Leitfähigkeitswert gemessen wird. Der entsprechende Wert wird dann von der Leitfähigkeitsmesseinrichtung 208 an ein Auswertegerät 211 weitergegeben, wo dann die Härte des Wassers bestimmt wird. Der Vorteil dieser Verfahrensvariante 200 besteht darin, dass nur eine Leitfähigkeitsmesseinrichtung 208 benötigt wird. Dadurch werden Nullpunktfehler eliminiert, was insbesondere eine deutlich genauere Bestimmung von Leitfähigkeitsdifferenzen ermöglicht. Die Bestimmung der Härte des Wassers ist immer noch in situ möglich. Kurzzeitige Schwankungen der Härte des Wassers sind aber nicht mehr detektierbar. Bei zeitlich langsamen Veränderungen der Wasserhärte, wie sie bei der öffentlichen Trinkwasserversorgung auftreten, ist dies jedoch nicht problematisch.

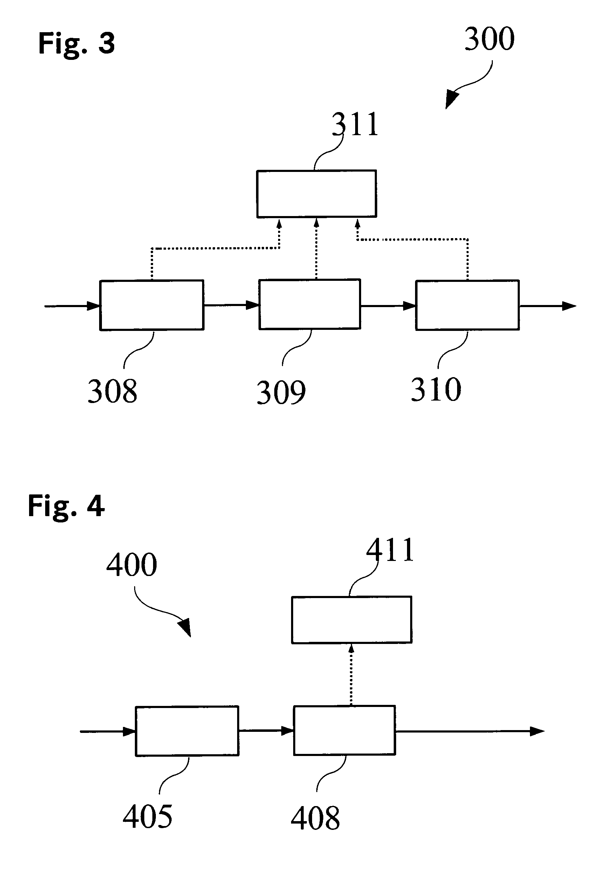

Die Figur 3 zeigt eine mögliche Abfolge der verschiedenen Schritte einer dritten erfindungsgemässen Verfahrensvariante 300. Bei dieser Verfahrensvariante 300 wird das Wasser, dessen Härte bestimmt werden soll, weder aufgeteilt noch durchläuft es einen Erhitzer oder eine andere Form einer Behandlungseinheit, sondern es wird direkt durch mehrere Messvorrichtungen 308, 309, 310 geleitet, die die elektrische Leitfähigkeit des Wassers bei jeweils unterschiedlichen Frequenzen messen, d. h. es werden zur Leitfähigkeitsbestimmung Wechselspannungen bzw. -ströme mit unterschiedlicher Schwingungsfrequenz eingesetzt.
Die Resultate der einzelnen Messungen werden dann in einem Auswertegerät 311 ausgewertet. Je nach Situation, das heisst falls mit Temperaturschwankungen des Wassers zu rechnen ist, kann eine Temperaturmesseinheit (nicht gezeichnet) vorhanden sein, deren Messung im Auswertegerät 311 ebenfalls berücksichtigt wird. Anstelle der Temperaturmesseinheit kann auch eine Regulierungseinheit (nicht gezeichnet) vorhanden sein, die sicherstellt, dass die Temperatur des Wassers konstant bleibt.
Die Figur 4 zeigt eine mögliche Abfolge der verschiedenen Schritte einer vierten erfindungsgemässen Verfahrensvariante 400. Wie in der Verfahrensvariante 300 wird auch in der vierten Verfahrensvariante 400 das zu messende Wasser nicht aufgeteilt. Im Unterschied zur dritten Verfahrensvariante 300, durchläuft das Wasser aber eine Behandlungseinrichtung 405 und danach eine Leitfähigkeitsmesseinrichtung 408. Indem die Behandlungseinrichtung 405 periodisch ein- respektive ausgeschaltet wird, durch läuft in zeitlichen Intervallen behandeltes und unbehandeltes Wasser die Leitfähigkeitsmesseinrichtung 408. Die Leitfähigkeitsmesseinrichtung 408 gibt die Werte an ein Auswertegerät 411 weiter, das dann die Härte des Wassers bestimmt. Alternativ, anstatt nur die Behandlungseinrichtung 405 ein- und auszuschalten, kann die Behandlungseinrichtung 405 ihre Behandlung zeitlich ändern. So kann zum Beispiel ein Erhitzer das Wasser auf unterschiedliche Temperaturen erhitzen. Dabei ist darauf zu achten, dass die entsprechende Temperatur auch lange genug gehalten wird, damit der entsprechende, zur dieser Temperatur gehörende Leitfähigkeitswert des Wassers gemessen werden kann.

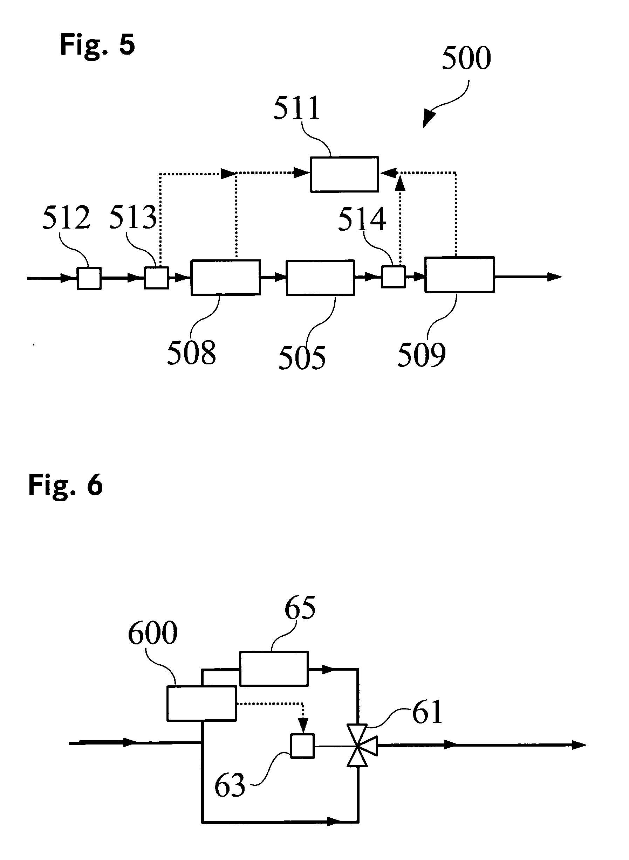

Die Figur 5 zeigt eine mögliche Abfolge der verschiedenen Schritte einer fünften erfindungsgemässen Verfahrensvariante 500. In einer ersten Messung 508 wird ein erster elektrischer Leitfähigkeitswert $\sigma_{CaH2O}$ von unbehandeltem Wasser gemessen. Dabei besteht der elektrische Leitfähigkeitswert $\sigma_{CaH2O}$ von unbehandeltem Wasser aus einem Anteil der elektrischen Leitfähigkeit $\sigma_{Ca}$ von Calcium-Ionen, welche im unbehandelten Wasser enthalten sind, und aus einem Anteil der elektrischen Leitfähigkeit $\sigma_{Rest}$ eines Restes weiterer Ionen im unbehandeltem Wasser. Danach erfolgt eine Entkalkung 505 des unbehandelten Wassers mittels eines Ionentauschers. Dabei werden die Calcium-Ionen durch Natrium-Ionen ersetzt. Danach wird in einer zweiten Messung 509 der elektrische Leitfähigkeitswert $\sigma_{NaH2O}$ des entkalkten Wassers bestimmt. Neben der elektrischen Leitfähigkeit des Rests der weiteren Ionen, der gleich gross ist wie bei der ersten Messung, tragen in der zweiten Messung nun anstelle der Calcium-Ionen die Natrium-Ionen zum elektrischen Leitfähigkeitswert $\sigma_{NaH2O}$ bei. Eine Differenz der elektrischen Leitfähigkeitswerten der ersten und der zweiten Messung $\sigma_{CaH2O} - \sigma_{NaH2O}$ entspricht daher einer Differenz der elektrischen Leitfähigkeitswerten $\sigma_{Ca}$ und $\sigma_{Na}$ der Calcium- und Natrium-Ionen, das heisst es gilt die Beziehung:

$$\sigma_{CaH2O} - \sigma_{NaH2O} = (\sigma_{Ca} + \sigma_{Rest}) - (\sigma_{Na} + \sigma_{Rest}) = \sigma_{Ca} - \sigma_{Na} \qquad (2)$$

Die elektrischen Leitfähigkeitswerte der Calcium-Ionen bzw. Natrium-Ionen werden im Wesentlichen durch die Beziehungen:

$$\sigma_{Ca} = \Lambda o_{Ca} * c_{Ca} \qquad (3)$$

und

$$\sigma_{Na} = \Lambda o_{Na} * c_{Na} \qquad (4)$$

bestimmt, wobei $\Lambda o_{Ca}$ die Grenzleitfähigkeit von Calcium bei 25°C ist und den Wert 59.5 [S*cm2/mol] hat und $\Lambda o_{Na}$ die Grenzleitfähigkeit von Natrium bei 25°C ist und den Wert 50.1 [S*cm$^2$/mol] hat. $c_{Ca}$ ist die Konzentration der Calcium-Ionen im unbehandelten Wasser und $c_{Na}$ die Konzentration der Natrium-Ionen, welche im Ionentauscher die Calcium-Ionen im unbehandelten Wasser ersetzt haben. Somit entspricht die Differenz der elektrischen Leitfähigkeitswerte $\sigma_{CaH2O} - \sigma_{NaH2O}$ dem Ausdruck:

$$\sigma_{CaH2O} - \sigma_{NaH2O} = (\Lambda o_{Na} * c_{Na} - \Lambda o_{Ca} * c_{Ca}) \qquad (5)$$

Aufgrund der unterschiedlichen Wertigkeit wird im Ionentauscher ein zweifachgeladenes Calcium-Ion durch zwei einfachgeladene Natrium-Ionen ersetzt, damit der Ionentauscher 505 elektrisch neutral bleibt. Somit ist die Konzentrationen $c_{Na}$ der Natrium-Ionen nach dem Ionentauscher 505 doppelt so gross, wie die Konzentration $c_{Na}$ der Calcium-Ionen vor dem Ionentauscher 505. Diese Tatsache berücksichtigend, kann die Beziehung (5) wie folgt geschrieben werden:

$$\sigma_{CaH2O} - \sigma_{NaH2O} = (2 * \Lambda o_{Na} * c_{Ca} - \Lambda o_{Ca} * c_{Ca}) \qquad (6)$$

Aufgelöst nach der Konzentration der Calcium-Ionen $c_{Ca}$ erhält man die Beziehung:

$$c_{Ca} = (\sigma_{NaH2O} - \sigma_{CaH2O}) / (2 * \Lambda o_{Na} - \Lambda o_{Ca}) \qquad (7)$$

Mit der Hilfe der Beziehung (7) wird aus den beiden elektrischen Leitungsfähigkeitswerten $\sigma_{CaH2O}$ und $\sigma_{NaH2O}$ dann in einer Auswertungseinheit 511 die Konzentration der Calcium-Ionen $c_{Ca}$ im unbehandelten Wasser bestimmt. In natürlich vorkommendem Wasser, insbesondere in Trinkwasser, ist der Beitrag der Magnesium-Ionen an der Härte von Wasser kleiner als 10% als jener von Calcium-Ionen. Somit kann der Beitrag der Magnesium-Ionen an der Härte von solchem Wasser vernachlässigt werden. Daher kann aus der Konzentration der Calcium-Ionen $c_{Ca}$ im unbehandelten Wasser zum Beispiel mit der Beziehung

$$1°fH = 0.2 * c_{Ca} \qquad (8)$$

die Härte des unbehandelten Wassers in französische Härtegrade berechnet werden, wobei die $c_{Ca}$ mit der Einheit $10^{-3}$ Mol pro Liter [mmol/l] bzw. Mol pro Kubik-Meter [mol/m$^3$] angegeben wird. Damit die Temperaturabhängigkeit der elektrischen Leitfähigkeit berücksichtigt werden kann, erfolgt vor der ersten Messung 508 der elektrischen Leitfähigkeit eine erste Temperaturmessung 513 und vor der zweiten Messung 509 der elektrischen Leitfähigkeit eine zweite Temperaturmessung 514. Die Werte der ersten und der zweiten Temperaturmessung 513, 514 werden dann durch die Auswertungseinheit 511 berücksichtigt.

Alternativ können die Messungen der Temperatur auch ein Heiz/Kühlgerät (in der Figur 5 nicht gezeichnet) steuern, welches das entkalkte Wasser für die zweite Messung 509 wieder auf die ursprüngliche Temperatur des unbehandelten Wassers der ersten Messung 508 bringt.

Je nach Verfahren, das zur Messung der elektrischen Leitfähigkeit angewandt wird, können Ablagerungen auf einer Messsonde Messwerte der ersten Messung 508 verfälschen. Damit dies nicht geschieht, erfolgt, wie dies in der Figur 5 gezeigt wird, eine Vorbehandlung 512 des unbehandelten Wassers, welche eine Ablagerung an der Messsonde minimiert, die erste Messung 508 der elektrische Leitfähigkeit des unbehandelten Wassers aber nicht beeinflusst. Eine solche Vorbehandlung 512 kann zum Beispiel mittels einer Biomineralisierung gemäss der Druckschrift EP 0 957 066 B1 oder physikalisch gemäss der Druckschrift EP 284 239 B1 erfolgen.

Die Figur 6 zeigt eine erste Ausführungsform 60 einer Verschnittwasseranlage, das heisst eine Anlage, die unbehandeltes Wasser mit weichem Wasser derart verschneidet, dass Wasser mit konstanter, optimaler Härte resultiert. Die Anlage beinhaltet neben einer Entkalkungsvorrichtung 65, zum Beispiel in Form eines Ionentauschers, ein steuerbares Mischventil 61, einen Regler 63 und eine Vorrichtung 600, die gemäss dem erfindungsgemässen Verfahren, insbesondere gemäss einer der Verfahrensvarianten 100, 200, 300, 400 oder 500, die Härte des Wassers bestimmt. In dieser ersten Ausführungsform 60 einer Verschnittwasseranlage erfolgt die Bestimmung der Härte des Wassers am unbehandelten Wasser vor der Entkalkungsvorrichtung 65. Die Bestimmung der Härte des unbehandelten Wassers steuert die Einstellung des Reglers 63 derart, dass nach dem Mischventil 61 das enthärtete Wasser einen konstanten Sollwert einer bestimmten Härte erreicht.

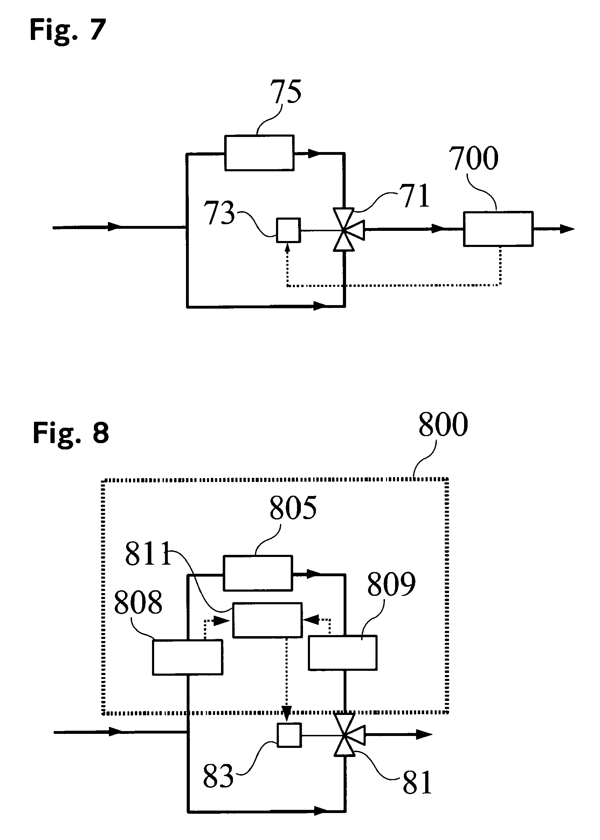

Die Figur 7 zeigt eine zweite Ausführungsform 70 einer Verschnittwasseranlage, wobei im Unterschied zur ersten Ausführungsform 60 eine Vorrichtung 700, die gemäss dem erfindungsgemässen Verfahren, insbesondere gemäss einer der Verfahrensvarianten 100, 200, 300, 400 oder 500, die Härte des Wassers bestimmt, erst nach einem Mischventil 71 angeordnet ist. Dies hat den Vorteil, dass nur Abweichungen vom Sollwert der Wasserhärte zu detektieren sind, mit anderen Worten, dass die Vorrichtung 72 zwecks einer optimalen Regelung nicht die allenfalls in weiten Grenzen schwankende Härte des unbehandelten Wassers messen muss. Ein weiterer Vorteil ist, dass mit der Anlage auch die Erschöpfung des Entkalkungsvorrichtung 75 detektiert werden kann. Vorausgesetzt, dass die Härte des unbehandelten Wassers über dem Sollwert der Härte des Mischwassers liegt, kündigt sich die Erschöpfung an oder ist bereits eingetreten, wenn das Mischventil 71 beinahe nur noch Wasser von der Entkalkungsvorrichtung 75 zudosiert oder ausschliesslich solches zudosiert.

Die Figur 8 zeigt eine dritte Ausführungsform 80 einer Verschnittwasseranlage. Ein Teil 800 der dritten Ausführungsform 80 entspricht im Wesentlichen einer Vorrichtung zum Bestimmen der Härte von Wasser nach der fünften Verfahrensvariante 500, wobei allfällige Berücksichtung der Temperaturdifferenzen und einer Vorbehandlung vor der ersten Messung nicht gezeichnet sind. Der Teil 800 der Ausführungsform 80 umfasst eine erste Messvorrichtung 808, eine zweite Messvorrichtung 809 und einen Ionentauscher 805 als Entkalkungsvorrichtung. Da nach der fünften Verfahrensvariante 500 am Ende entkalktes Wasser vorhanden ist, kann mit einem Mischventil 81 dieses entkalkte Wasser mit unbehandeltem Wasser verschnitten werden. Dabei wird das Mischventil 81 durch einen Regler 83 gesteuert. Dieser stellt ein Mischverhältnis des unbehandelten und des entkalkten Wassers derart ein, dass ein vorgegebener Sollwert des enthärteten Wassers nach dem Mischventil 81 erhalten wird. Dazu benutzt der Regler 83 die Werte der Härte vom unbehandelten Wasser, welche mittels des Teils 800 bestimmt wurden.

Im Allgemeinen können Messvorrichtungen, die einen Wechselstrom anlegen, so ausgestattet sein, dass dieselbe Messvorrichtung die elektrische Leitfähigkeit für zwei verschiedene Frequenzen misst. Dabei kann eine Messeinrichtung bei mehreren Frequenzen messen oder es ist auch möglich, dass mehrere Messvorrichtungen vorhanden sind, die mit Hilfe von Wechselstrom die elektrische Leitfähigkeit für verschiedene Frequenzen gleichzeitig messen. Als Frequenzbereiche kommt insbesondere der Bereich von 1 kHz bis 100 MHz, vorteilhaft von 10 kHz bis 200 kHz in Frage. Frequenzen darüber und darunter sind aber ebenfalls möglich.

Pro Teilstrom, respektive Fraktion der Wasserprobe können sowohl einer als auch mehrere Werte der elektrischen Leitfähigkeit gemessen werden. Dabei sollten die verschiedenen Messprozesse am gleichen Teilstrom unterschiedlich sein. Dies ist zum Beispiel dann gegeben, wenn mehrere Messungen mit der Hilfe von Wechselströmen unterschiedlicher Frequenzen durchgeführt werden.

Die einzelnen Messvorrichtungen respektive Verfahren zum Messen der elektrischen Leitfähigkeit können auf den in ihrem Teilstrom zu erwartenden Messbereich optimiert werden. So ist bei höher erhitzten Teilströmen respektive Fraktionen eine geringere IonenKonzentration zu erwarten. Daher werden die Messwerte kleiner ausfallen als für weniger erhitzte Teilströme.

Die Temperatur beim Erhitzen zur Behandlung der einzelnen Fraktionen kann sich grundsätzlich über den ganzen Bereich erstrecken, bei dem Wasser beim jeweils herrschenden Druck flüssig ist. Damit die Wasserprobe nicht gekühlt werden muss und somit eine teure Kühlvorrichtung vorhanden sein muss, ist eine untere Temperatur von 20°C vorteilhaft.

Die einzelnen Temperaturen, auf welche die Fraktionen erhitzt werden, sollten so gewählt werden, dass ein deutlicher Unterschied in der elektrischen Leitfähigkeit nach der Behandlung messbar ist.

Es ist möglich, dass nur eine der Messvorrichtungen den absoluten Wert der elektrischen Leitfähigkeit misst und die anderen Messvorrichtungen an ihren Fraktionen nur noch eine Differenzmessung zur absoluten Messung durchführen.

Es sind auch Kombinationen der verschiedenen Verfahrensvarianten vorstellbar. So kann zum Beispiel die erste Verfahrensvariante 100 mit der vierten Verfahrensvariante 400 kombiniert werden. So können die Behandlungseinrichtungen 105, 106 und 107 der ersten Verfahrensvariante 100 wie bei der vierten Verfahrensvariante 400 periodisch ein- und ausgeschaltet respektive deren Aktivität verändert werden. Die Leitfähigkeitsmesseinrichtungen 108, 109 und 110 geben dann wie die Leitfähigkeitsmesseinrichtung 408 ihre zeitlich aufgelösten Werte an das Auswertegerät weiter. Durch eine solche Kombination kann die Anzahl Messwerte des Wassers unter verschiedenen Bedingungen erhöht werden, ohne dass zusätzliche Behandlungseinrichtungen, respektive Leitfähigkeitsmesseinrichtungen gebraucht würden.

Unter Ionentauscher sind alle Vorrichtungen zu verstehen, die Wasser Calcium-Ionen entziehen und gegebenenfalls durch andere Ionen ersetzen. In der Verfahrensvariante 500 wurde davon ausgegangen, dass der Ionentauscher die Calcium-Ionen durch Natrium- Ionen ersetzt. Falls der Ionentauscher anstelle von Natrium andere Ionen dem Wasser zuführt, die in irgendeiner Weise die elektrische Leitfähigkeit beeinflussen, oder auch keine Ionen zuführt, müssen die Beziehungen (1) bis (7) entsprechend angepasst werden, insbesondere sind Werte der Grenzleitfähigkeit und unterschiedliche Wertigkeiten anzupassen.

Zur Messung der elektrischen Leitfähigkeit von Wasser können Messvorrichtungen verwendet werden, die eine Temperaturkorrektur automatisch vornehmen und einen bezüglich der Temperatur normierten elektrischen Leitfähigkeitswert als Ausgangssignal haben. In diesem Falle erübrigen sich Temperaturmessungen, wie sie in der Figur 5 dargestellt wurden.

Die Änderung der elektrischen Leitfähigkeit aufgrund der Änderung der Temperatur ist in einer ersten Näherung für unterschiedliche Ionen- Konzentrationen in Wasser gleich. Daher ist es möglich, nur die Temperaturdifferenz bei den einzelnen Messungen zu berücksichtigen. Sind grosse Temperaturdifferenzen zu erwarten bzw. ist die Messgenauigkeit zu verbessern, so muss die Temperaturabhängigkeit der elektrischen Leitfähigkeit gemäss der erwähnten DIN-Norm DIN EN 27888 : 1993 berücksichtigt werden.

Zusammenfassend ist festzustellen, dass durch die Erfindung ein einfaches Verfahren sowie eine Vorrichtung geschaffen werden, mit deren Hilfe kontinuierlich die Härte von Wasser bestimmt werden kann. Dabei wird weitgehend auf den Zusatz von Chemikalien verzichtet, was zu einer wartungsarmen Vorrichtung führt.

**Patentansprüche**

1. Verfahren zur Bestimmung der Härte von Wasser, **dadurch gekennzeichnet, dass** mit einer Wasserprobe folgende Schritte ausgeführt werden:

a) Bestimmen mindestens zweier Werte der elektrischen Leitfähigkeit der Wasserprobe unter verschiedenen Bedingungen und

b) Bestimmen der Härte des Wassers der Wasserprobe aus den mindestens zwei Werten der elektrischen Leitfähigkeit.

2. Verfahren nach Anspruch 1, **dadurch gekennzeichnet, dass** vorgängig folgende zwei Schritte ausgeführt werden:

a) Aufteilen der Wasserprobe in mindestens zwei Fraktionen (102, 103, 104; 202, 203, 204),

b) Unterziehen mindestens einer der Fraktionen einer Behandlung, welche die elektrische Leitfähigkeit des Wassers beeinflussen kann,

wobei die Bestimmung der Werte der elektrischen Leitfähigkeit des Wassers an mindestens zwei der Fraktionen (102, 103, 104; 202, 203, 204) vorgenommen wird.

3. Verfahren nach Anspruch 2, **dadurch gekennzeichnet, dass** mindestens eine der Fraktionen (102, 103, 104; 202, 203, 204) unbehandelt bleibt.

4. Verfahren nach einem der Ansprüche 2 bis 4, **dadurch gekennzeichnet, dass** die Behandlung mindestens einer der Fraktionen (102, 103, 104; 202, 203, 204) chemisch, insbesondere elektro-chemisch oder katalytisch, erfolgt.

5. Verfahren nach Anspruch 2 oder 3, **dadurch gekennzeichnet, dass** die Behandlung mindestens einer der Fraktionen (102, 103, 104; 202, 203, 204) physikalisch, insbesondere durch Erhitzen, erfolgt.

6. Verfahren nach einem der Ansprüche 1 bis 4, **dadurch gekennzeichnet, dass** eine erste Messung (508; 809) einen elektrischen Leitfähigkeitswert $\sigma_{CaH2O}$ von unbehandeltem Wasser bestimmt, das unbehandelte Wasser mittels eines Ionentauschers (505; 805) behandelt wird, um entkalktes behandeltes Wasser zu erhalten, eine zweite Messung (509; 809) einen elektrischen Leitfähigkeitswert $\sigma_{NaH2O}$ von entkalktem Wasser nach dem Ionentauscher bestimmt.

7. Verfahren nach Anspruch 6, **dadurch gekennzeichnet, dass** aus den bestimmten elektrischen Leitfähigkeitswerten $\sigma_{CaH2O}$ und $\sigma_{NaH2O}$ mittels einer Beziehung

$$c_{Ca}=(\sigma_{NaH2O}-\sigma_{CaH2O})/(2*\Lambda o_{Na}-\Lambda o_{Ca})$$

eine Konzentration $c_{Ca}$ von Calcium-Ionen des unbehandelten Wassers ermittelt wird,

wobei $\Lambda o_{Ca}$ die Grenzleitfähigkeit von Calcium bei 25°C ist und $\Lambda o_{Na}$ die Grenzleitfähigkeit von Natrium bei 25°C ist.

8. Verfahren nach Anspruch 7, **dadurch gekennzeichnet, dass** eine Wassertemperaturdifferenz zwischen der ersten Messung (508; 808) und der zweiten Messung (509; 809) berücksichtigt wird, indem der gemessene elektrische Leitfähigkeitwert $\sigma_{CaH2O}$ von unbehandeltem Wasser und/oder der gemessene elektrische Leitfähigkeitswert $\sigma_{NaH2O}$ von entkalktem Wasser nach dem Ionentauscher (505, 805) entsprechend korrigiert wird .

9. Verfahren nach Anspruch 7, **dadurch gekennzeichnet, dass** mittels einer Temperaturänderungsvorrichtung eine Temperatur des entkalkten Wassers derart verändert wird, dass die Temperatur des entkalkten Wassers während der zweiten Messung einer Temperatur des unbehandelten Wassers entspricht.

10. Verfahren nach einem der Ansprüche 2 bis 9, **dadurch gekennzeichnet, dass** Ablagerungen, die infolge einer Wasserbehandlung entstehen, mittels entsprechender Methoden, insbesondere mit Ultraschall, periodisch und automatisch entfernt werden.

11. Verfahren nach einem der Ansprüche 3 bis 9, **dadurch gekennzeichnet, dass** Ablagerungen, die infolge einer Wasserbehandlung entstehen, durch permanente Anwendung entsprechender Methoden, insbesondere mit Ultraschall, verhindert werden.

12. Verfahren nach einem der Ansprüche 6 bis 12, **dadurch gekennzeichnet, dass** das zu messende unbehandelte Wasser einer Vorbehandlung unterzogen wird, so dass eine Ablagerung an einer Messsonde zur Messung der elektrischen Leitfähigkeit minimiert wird, ohne dass die Härte des unbehandelte Wassers verändert wird.

13. Verfahren nach einem der Ansprüche 1 bis 12, **dadurch gekennzeichnet, dass** zur Bestimmung der mindestens zwei Werte der elektrischen Leitfähigkeit Messungen mithilfe von einem Wechselsignal, insbesondere einem Wechselstrom, bei verschiedenen Frequenzen vorgenommen werden.

14. Verfahren zum Enthärten von Wasser, wobei unbehandeltes Wasser zuerst entkalkt wird und danach mit unbehandeltem Wasser zu enthärtetem Wasser verschnitten wird, **dadurch gekennzeichnet, dass** in zeitlichen Abständen eine Härte des unbehandelten Wassers nach einem der Ansprüche 1 bis 13 bestimmt wird und dass mittels der bestimmten Härte ein Mischverhältnis von unbehandeltem Wasser und entkalktem Wasser derart eingestellt wird, dass eine bestimmte Härte des enthärteten Wassers erhalten wird.

15. Vorrichtung zur Bestimmung der Härte des Wassers eines Wasserstroms nach einem Verfahren nach einem der Ansprüche 1 bis 13, bestehend aus:

   a) mindestens einer Messvorrichtung (108, 109, 110; 208, 209, 210; 308, 309, 310; 408; 508, 509; 805, 809) zum Messen der elektrischen Leitfähigkeit des Wasserstroms,
   b) einer Auswertungseinheit (111; 211; 311; 411; 511; 811) zum Auswerten von mindestens zwei, unter verschiedenen Bedingungen vorgenommenen Messungen der elektrischen Leitfähigkeit zum Bestimmen der Härte des Wassers.

16. Vorrichtung nach Anspruch 15, **dadurch gekennzeichnet, dass** zusätzlich:

   a) eine Verteilstation (101) zum Trennen des Wasserstroms in mindestens zwei Teilströme (102, 103, 104; 202, 203, 204),
   b) mindestens eine Behandlungsvorrichtung (105, 106, 107; 205, 206, 207; 505; 805) zur Beeinflussung der elektrischen Leitfähigkeit des Wassers eines Teilstroms, insbesondere ein kontinuierlicher Reaktor (Konti-Reaktor) und
   c) mehrere Messvorrichtungen (108, 109, 110; 208, 209, 210) zum Messen der Leitfähigkeit der Teilströme (102, 103, 104; 202, 203, 204)
   vorhanden sind.

17. Vorrichtung nach einem der Ansprüche 15 oder 16, **dadurch gekennzeichnet, das** eine erste Messvorrichtung zur Messung (508; 808) der elektrischen Leitfähigkeit von unbehandeltem Wasser, ein Ionentauscher zur Behandlung von Wasser und eine zweite Messvorrichtung zur Messung (509; 809) der elektrischen Leitfähigkeit von behandeltem Wasser nach dem Ionentauscher (505; 805) vorhanden sind.

18. Vorrichtung nach einem der Ansprüche 15 bis 17, **dadurch gekennzeichnet, dass** Reinigungseinheiten zum Entfernen von Ablagerungen, insbesondere von Ablagerungen in den Behandlungseinheiten, vorhanden sind.

19. Vorrichtung nach Anspruch 18, **dadurch gekennzeichnet, dass** die Reinigungseinheiten ein Ultraschallgerät beinhalten.

20. Vorrichtung nach einem der Ansprüche 15 bis 19, **dadurch gekennzeichnet, dass** die Messvorrichtung (108, 109, 110; 208, 209, 210; 308, 309, 310; 410; 508, 509; 805, 809) eine Einrichtung zur Erzeugung von Wechselsignalen, insbesondere Wechselströmen, voneinander verschiedener Frequenzen umfasst und dass sie derart ausgebildet ist, dass die Leitfähigkeit bei den verschiedenen Frequenzen messbar ist.

21. Anordnung zum Enthärten von Wasser, welche eine Entkalkungsvorrichtung (805) zum Entkalken von Wasser, eine Verschnittvorrichtung (61; 71; 81) zum Verschneiden von unbehandeltem Wasser mit entkalktem Wasser zu enthärtetem Wasser und eine Vorrichtung zur Bestimmung der Härte von unbehandeltem Wassers (100; 200; 300; 400; 500; 600; 700; 800) nach einem der Ansprüche 16 bis 21 umfasst, **dadurch gekennzeichnet, dass** die Verschnittvorrichtung (61; 71; 81) mittels der Vorrichtung zur Bestimmung der Härte des unbehandelten Wassers derart eingestellt wird, dass eine bestimmte Härte des enthärteten Wassers erhalten wird.

**Fig. 1**

**Fig. 2**

**Fig. 3**

300

311

308      309      310

**Fig. 4**

400

411

405      408

**Fig. 5**

500

511

512  513  514

508  505  509

**Fig. 6**

600  65

61

63

**Fig. 7**

**Fig. 8**

Europäisches
Patentamt

European
Patent Office

Office européen
des brevets

**EUROPÄISCHER RECHERCHENBERICHT**

**Nummer der Anmeldung**

EP 09 40 5166

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 41 14 380 A1 (DAIMLER BENZ AG [DE]) 5. November 1992 (1992-11-05) | 1-4,6-9, 15-17 | INV. G01N27/08 |
| Y | * Seite 2, Zeilen 29-43 * | 10-12, 18,19,21 | G01N33/18 |
| | * Seite 2, Zeilen 60-64 * * Seite 3, Zeilen 9-12 * * Seite 3, Zeilen 40-47; Abbildung 1 * ----- | | |
| X | US 4 801 551 A (BYERS WILLIAM A [US] ET AL) 31. Januar 1989 (1989-01-31) | 1-3,5, 15,16 | |
| Y | * Spalte 1, Zeile 63 - Spalte 2, Zeile 4 * | 10-12, 18,19,21 | |
| | * Spalte 2, Zeile 58 - Spalte 3, Zeile 4 * * Spalte 3, Zeilen 49-54 * * Spalte 5, Zeilen 3-42; Abbildung 5 * ----- | | |
| X | US 2007/024287 A1 (GRAVES PARKER P [US] ET AL) 1. Februar 2007 (2007-02-01) | 1-3,13, 20 | |
| Y | * Absätze [0003], [0006], [0026], [0029], [0034], [0035] * | 10,11, 14,18, 19,21 | |
| | ----- | | RECHERCHIERTE SACHGEBIETE (IPC) |
| X | EP 0 274 396 A (HITACHI LTD [JP]) 13. Juli 1988 (1988-07-13) * Seite 2, Zeile 43 - Seite 3, Zeile 16 * ----- | 1-3,5, 15,16 | G01N |
| X | US 7 250 775 B1 (COLLINS JOHN [US] ET AL) 31. Juli 2007 (2007-07-31) * Spalte 2, Zeilen 20-39 * * Spalte 8, Zeilen 45-55 * * Ansprüche 1,11 * ----- | 1,13,15, 20 | |
| X,D | DE 198 26 659 A1 (WITTMANN JUERGEN [DE]) 23. Dezember 1999 (1999-12-23) * das ganze Dokument * ----- | 1,15 | |
| | -/-- | | |

Der vorliegende Recherchenbericht wurde für alle Patentansprüche erstellt

| Recherchenort | Abschlußdatum der Recherche | Prüfer |
|---|---|---|
| München | 2. Dezember 2009 | Stussi, Elisa |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

.....................................................................

& : Mitglied der gleichen Patentfamilie, übereinstimmendes
    Dokument

EPO FORM 1503 03.82 (P04C03)

| Europäisches Patentamt European Patent Office Office européen des brevets | **EUROPÄISCHER RECHERCHENBERICHT** | **Nummer der Anmeldung** EP 09 40 5166 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | FR 2 834 509 A (ELECTRICITE DE FRANCE [FR]) 11. Juli 2003 (2003-07-11) * Zusammenfassung * ----- | 10-12, 18,19 | |
| Y | US 2007/215531 A1 (WAWRLA ANDREAS [CH] ET AL) 20. September 2007 (2007-09-20) * Zusammenfassung * ----- | 14,21 | |
| A,D | DE 195 37 059 A1 (UNIV DRESDEN TECH [DE]) 10. April 1997 (1997-04-10) * das ganze Dokument * ----- | 1-21 | |
| A | MARTINHO E ET AL: "An experimental study of organic pollutant effects on time domain induced polarization measurements" JOURNAL OF APPLIED GEOPHYSICS, ELSEVIER, AMSTERDAM, NL, Bd. 60, Nr. 1, 1. September 2006 (2006-09-01), Seiten 27-40, XP025142654 ISSN: 0926-9851 [gefunden am 2006-09-01] * das ganze Dokument * ----- | 1-21 | RECHERCHIERTE SACHGEBIETE (IPC) |

Der vorliegende Recherchenbericht wurde für alle Patentansprüche erstellt

| Recherchenort München | Abschlußdatum der Recherche 2. Dezember 2009 | Prüfer Stussi, Elisa |
|---|---|---|

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..............................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 09 40 5166

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-12-2009

| Im Recherchenbericht angeführtes Patentdokument | | Datum der Veröffentlichung | Mitglied(er) der Patentfamilie | | Datum der Veröffentlichung |
|---|---|---|---|---|---|
| DE 4114380 | A1 | 05-11-1992 | KEINE | | |
| US 4801551 | A | 31-01-1989 | KEINE | | |
| US 2007024287 | A1 | 01-02-2007 | KEINE | | |
| EP 0274396 | A | 13-07-1988 | CN | 88100066 A | 23-11-1988 |
| | | | DE | 3884971 D1 | 25-11-1993 |
| | | | DE | 3884971 T2 | 31-03-1994 |
| | | | JP | 5041943 B | 25-06-1993 |
| | | | US | 4853638 A | 01-08-1989 |
| US 7250775 | B1 | 31-07-2007 | KEINE | | |
| DE 19826659 | A1 | 23-12-1999 | KEINE | | |
| FR 2834509 | A | 11-07-2003 | KEINE | | |
| US 2007215531 | A1 | 20-09-2007 | EP | 1834927 A1 | 19-09-2007 |
| DE 19537059 | A1 | 10-04-1997 | KEINE | | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19854651
- DE 3406724
- DE 19537059 A1
- DE 4114380 A1
- DE 19826659 A1

- DE 102007002016 A1
- EP 0957066 A1
- EP 0957066 B1
- EP 284239 B1